# EUROPEAN PATENT APPLICATION

(11) **EP 1 616 567 A1**
(43) Date of publication of application: **18.01.2006**
(21) Application number: 04016878.3
(22) Date of filing: 16.07.2004
(51) Int. Cl.: A61K 31/4025, A61K 45/06, A61P 11/00, A61K 31/192, A61K 31/44

(54) **Medicaments for inhalation comprising PDE IV inhibitors and glycopyrrolate salts**

(71) Applicant: Boehringer Ingelheim Pharma GmbH & Co.KG, 55216 Ingelheim am Rhein (DE)
(72) Inventor: The designation of the inventor has not yet been filed

(57) **Abstract**

The present invention relates to novel pharmaceutical compositions based on PDE IV inhibitors **2** and salts of formula **1** 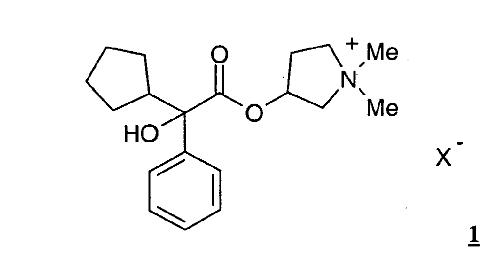 wherein X⁻ may have the meanings defined in the description and claims, processes for preparing them and their use in the treatment of respiratory complaints.

## Description

The present invention relates to novel pharmaceutical compositions based on PDE IV inhibitors **2** and salts of formula **1** 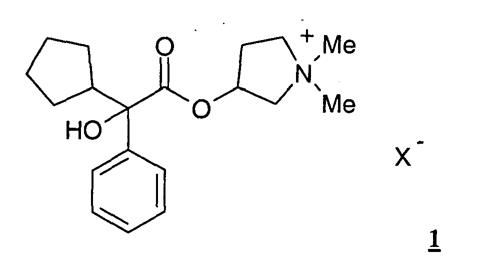
wherein X- may have the meanings defined in the description and claims, processes for preparing them and their use in the treatment of respiratory complaints.

### Description of the invention

The present invention relates to novel pharmaceutical compositions based on PDE IV inhibitors **2** and salts of formula **1**, processes for preparing them and their use in the treatment of respiratory complaints.

Accordingly, the invention is directed to pharmaceutical compositions comprising one or more, preferably one salt of formula **1** 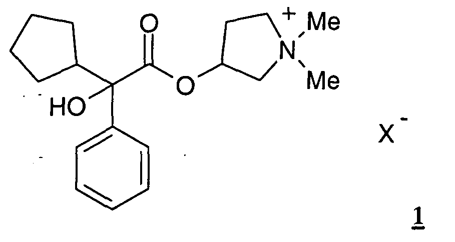
wherein
- X -: denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate, optionally in the form of the racemates, the stereoisomers, and the hydrates thereof,
in combination with one or more, preferably one PDE IV inhibitor (**2**), optionally together with a pharmaceutically acceptable excipient.

Preferably, the salts of formula **1** are used wherein
- X⁻: denotes an anion with a single negative charge selected from among the fluoride, chloride, bromide, 4-toluenesulphonate and methanesulphonate, preferably bromide, optionally in the form of the racemates, the stereoisomers, and the hydrates thereof.

Most preferably, the salts of formula **1** are used wherein
- X⁻: denotes an anion with a single negative charge selected from among the chloride, bromide and methanesulphonate, preferably bromide, optionally in the form of the racemates, the stereoisomers, and the hydrates thereof.

Particularly preferred according to the invention is the salt of formula **1** wherein X - denotes bromide.

The compounds of formula **1** may be present in form of their racemates, in form of their diastereomers or in form of mixtures of their diastereomers.

Of particular interest according to the invention are pharmaceutical compositions according to the invention comprising the diastereoisomers of formula **(2R',3S) 1** 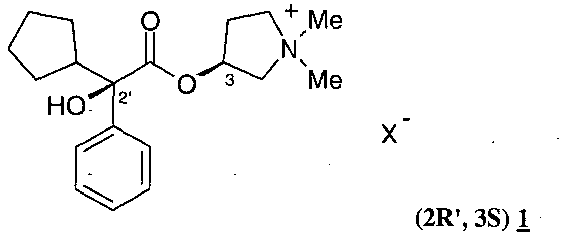 wherein X - may have the meanings an mentioned hereinbefore.

In another preferred embodiment pharmaceutical compositions according to the invention are comprising the diastereoisomers of formula **(2S',3S) 1** 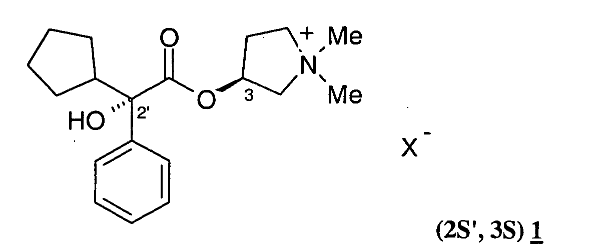
wherein X - may have the meanings an mentioned hereinbefore.

In a yet another preferred embodiment pharmaceutical compositions according to the invention are comprising the diastereoisomers of formula **(2S',3R) 1** 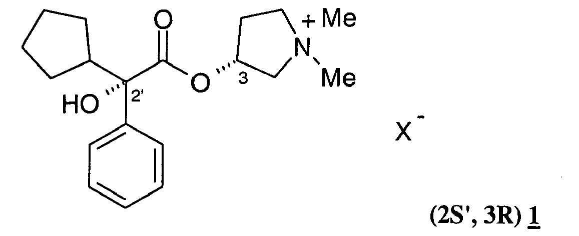
wherein X - may have the meanings an mentioned hereinbefore.

Of particular interest and accordingly particularly preferred are pharmaceutical compositions according to the invention comprising the diastereoisomers of formula **(2R',3R) 1** 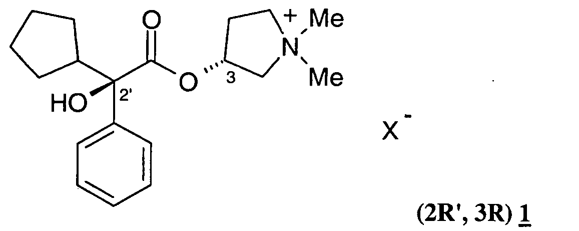 wherein X - may have the meanings an mentioned hereinbefore.

Surprisingly, an unexpectedly beneficial therapeutic effect can be observed in the treatment of inflammatory and/or obstructive diseases of the respiratory tract if the anticholinergic of formula **1** is used with one or more, preferably one PDE IV inhibitor **2**.

The beneficial therapeutic effect mentioned above may be observed both when the two active substances are administered simultaneously in a single active substance formulation and when they are administered successively in separate formulations. According to the invention, it is preferable to administer the two active substance ingredients simultaneously in a single formulation.

In the pharmaceutical combinations mentioned above the active substances may be combined in a single preparation or contained in two separate formulations. Pharmaceutical compositions which contain the active substances **1** and **2** in a single preparation are preferred according to the invention.

According to the instant invention preferred PDE IV inhibitors **2** in the combinations according to the invention are selected from the group consisting of enprofylline, theophylline, roflumilast, ariflo (cilomilast), CP-325,366, BY343, D-4396 (Sch-351591), AWD-12-281 (GW-842470), N-(3,5-dichloro-1-oxo-pyridin-yl)-4-difluoromethoxy-3-cyclopropylmethoxybenzamide, NCS-613, pumafentine, (-)p-[(4*a*R*,10*b*S*)-9-ethoxy-1,2,3,4,4a,10b-hexahydro-8-methoxy-2methylbenzo[s][1,6]naphthyridin-6-yl]-N,N-diisopropylbenzamide, (R)-(+)-1-(4-bromobenzyl)-4-[(3-cyclopentyloxy)-4-methoxyphenyl]-2-pyrrolidone, 3-(cyclopentyloxy-4-methoxyphenyl)-1-(4-N'-[N-2-cyano-S-methyl-isothioureido]benzyl)-2-pyrrolidone, cis[4-cyano-4-(3-cyclopentyloxy-4-methoxyphenyl)cyclohexan-1-carboxylic acid], 2-carbomethoxy-4-cyano-4-(3-cyclopropylmethoxy-4-difluoromethoxyphenyl)cyclohexan-1-one cis[4-cyano-4-(3-cyclopropylmethoxy-4-difluoromethoxyphenyl)cyclohexan-1-ol], (R)-(+)-ethyl[4-(3-cyclopentyloxy-4-methoxyphenyl)pyrrolidine-2-ylidene]acetate, (S)-(-)-ethyl[4-(3-cyclopentyloxy-4-methoxyphenyl)pyrrolidine-2-ylidene]acetate, CDP840, Bay-198004, D-4418, PD-168787, T-440, T-2585, arofylline, atizoram, V-11294A, Cl-1018, CDC-801, CDC-3052, D-22888, YM-58997, Z-15370, 9-cyclopentyl-5,6-dihydro-7-ethyl-3-(2-thienyl)-9*H*-pyrazolo[3,4-c]-1,2,4-triazolo[4,3-a]pyridine, and 9-cyclopentyl-5,6-dihydro-7-ethyl-3-(*tert*-butyl)-9*H*-pyrazolo[3,4-c]-1,2,4-triazolo[4,3-a]pyridine, optionally in the form of the racemates, the enantiomers, the diastereomers and optionally the pharmacologically acceptable acid addition salts thereof, and the hydrates thereof.

In a preferred embodiment according to the invention the PDE IV inhibitors **2** are selected from the group consisting of enprofylline, roflumilast, ariflo (cilomilast), AWD-12-281 (GW-842470), N-(3,5-dichloro-1-oxo-pyridin-4-yl)-4-difluoromethoxy-3-cyclopropylmethoxybenzamide, T-440, T-2585, arofylline, cis[4-cyano-4-(3-cyclopentyloxy-4-methoxyphenyl)cyclohexan-1-carboxylic acid], 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-l-ol], PD-168787, atizoram, V-11294A, C1-1018, CDC-801, D-22888, YM-58997, Z-15370, 9-cyclopentyl-5,6-dihydro-7-ethyl-3-(2-thienyl)-*9H*-pyrazolo[3,4-c]-1,2,4-triazolo[4,3-a]pyridine, and 9-cyclopentyl-5,6-dihydro-7-ethyl-3-(*tert*-butyl)-9*H*-pyrazolo[3,4-c]-1,2,4-triazolo[4,3-a]pyridine, optionally in the form of the racemates, the enantiomers, the diastereomers and optionally the pharmacologically acceptable acid addition salts thereof, and the hydrates thereof.

In another preferred embodiment according to the invention the PDE IV inhibitors **2** are selected from the group consisting of roflumilast, ariflo (cilomilast), AWD-12-281 (GW-842470), arofylline, Z-15370, 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], atizoram, 9-cyclopentyl-5,6-dihydro-7-ethyl-3-(2-thienyl)-9*H*-pyrazolo[3,4-c]-1,2,4-triazolo[4,3-a]pyridine, and 9-cyclopentyl-5,6-dihydro-7-ethyl-3-(tert-butyl)-9*H*-pyrazolo[3,4-c]-1,2,4-triazolo[4,3-a]pyridine, while roflumilast, Z-15370, and AWD-12-281 are particularly preferred as compound **2** according to the invention.

Pharmaceutically acceptable salt forms of the combinations of compounds of the present invention are prepared for the most part by conventional means. Where the component compound contains a carboxylic acid group, a suitable salt thereof may be formed by reacting the compound with an appropriate base to provide the corresponding base addition salt. Examples of such bases are alkali metal hydroxides including potassium hydroxide, sodium hydroxide, and lithium hydroxide; alkaline earth metal hydroxides such as barium hydroxide and calcium hydroxide; alkali metal alkoxides, *e*.*g*., potassium ethanolate and sodium propanolate; and various organic bases such as piperidine, diethanolamine, and *N-*methylglutamine. Also included are the aluminum salts of the component compounds of the present invention.

For certain component compounds acid addition salts may be formed by treating said compounds with pharmaceutically acceptable organic and inorganic acids, *e.g.*, hydrohalides such as hydrochloride, hydrobromide, hydroiodide; other mineral acids and their corresponding salts such as sulfate, nitrate, phosphate, *etc.*; and alkyl- and mono-arylsulfonates such as ethanesulfonate, toluenesulfonate, and benzenesulfonate; and other organic acids and their corresponding salts such as acetate, tartrate, maleate, succinate, citrate, benzoate, salicylate, ascorbate, etc.

Accordingly, the pharmaceutically acceptable acid addition salts of the component compounds of the present invention include, but are not limited to: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate, phthalate.

Particularly preferred examples of pharmacologically acceptable acid addition salts of the compounds **2** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid or maleic acid. If desired, mixtures of the abovementioned acids may also be used to prepare the salts **2**.

In the pharmaceutical compositions according to the invention, the compounds **2** may be present in the form of their racemates, enantiomers or mixtures thereof. The separation of the enantiomers from the racemates may be carried out using methods known in the art (e.g. by chromatography on chiral phases, etc.).

In one aspect the present invention relates to the abovementioned pharmaceutical compositions which contain, in addition to therapeutically effective quantities of **1** and **2**, a pharmaceutically acceptable carrier. In another aspect the present invention relates to the abovementioned pharmaceutical compositions which do not contain any pharmaceutically acceptable carrier in addition to therapeutically effective quantities of **1** and **2**.

The present invention also relates to the use of therapeutically effective quantities of the salts **1** for preparing a pharmaceutical composition containing PDE IV inhibitors **2** for treating inflammatory or obstructive diseases of the respiratory tract. Preferably, the present invention relates to the abovementioned use for preparing a pharmaceutical composition for treating asthma or COPD.

Within the scope of the present invention the compounds **1** and **2** may be administered simultaneously or successively, while it is preferable according to the invention to administer compounds **1** und **2** simultaneously.

The present invention further relates to the use of therapeutically effect amounts of salts **1** and PDEIV inhibitors **2** for treating inflammatory or obstructive respiratory complaints, particularly asthma or COPD.

The proportions in which the active substances **1** and **2** may be used in the active substance combinations according to the invention are variable. Active substances **1** and **2** may possibly be present in the form of their solvates or hydrates. Depending on the choice of the compounds **1** and **2**, the weight ratios which may be used within the scope of the present invention vary on the basis of the different molecular weights of the various salt forms.

As a rule, the pharmaceutical combinations according to the invention may contain compounds **1** and **2** in ratios by weight ranging from 1:100 to 100:1, preferably from 1:80 to 80:1. In particularly preferred pharmaceutical combinations that contain as component **2** one of the most preferred compounds ariflo, roflumilast, or AWD-12-281, the weight ratios of **1** to **2** are most preferably in a range in which **1** and **2** are present in proportions of 1:50 to 50:1, more preferably from 1:30 to 30:1.

For example, without restricting the scope of the invention thereto, preferred combinations of **1** and PDE-IV inhibitor **2** (as for instance ariflo, roflumilast or AWD-12-281) may contain in the following weight ratios: 1:65, 1:64, 1:63, 1:62, 1:61, 1:60, 1:59, 1:58, 1:57, 1:56, 1:55, 1:54, 1:53, 1:52, 1:51, 1:50; 1:49; 1:48; 1:47; 1:46; 1:45; 1:44; 1:43; 1:42; 1:41; 1:40; 1:39; 1:38; 1:37; 1:36; 1:35; 1:34; 1:33; 1:32; 1:31; 1:30; 1:29; 1:28; 1:27; 1:26; 1:25; 1:24; 1:23; 1:22; 1:21; 1:20; 1:19; 1:18; 1:17; 1:16; 1:15; 1:14; 1:13; 1:12; 1:11; 1:10; 1:9; 1:8; 1:7; 1:6; 1:5; 1:4; 1:3; 1:2; 1:1; 2:1; 3:1; 4:1; 5:1; 6:1; 7:1; 8:1; 9:1; 10:1; 11:1; 12:1; 13:1; 14:1; 15:1; 16:1; 17:1; 18:1; 19:1; 20:1; 21:1; 22:1; 23:1; 24:1; 25:1; 26:1; 27:1; 28:1; 29:1; 30 :1.

The pharmaceutical compositions according to the invention containing the combinations of **1** and **2** are normally administered so that **1** and **2** are present together in doses of 0.1 to 10000µg, preferably from 1 to 5000µg, more preferably from 10 to 3000µg, better still from 100 to 2000µg per single dose. For example, combinations of **1** and **2** according to the invention contain a quantity of **1** and PDE-IV inhibitor **2** (as for instance ariflo, roflumilast or AWD-12-281) such that the total dosage per single dose is about 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg, 1000µg, 1005µg, 1010µg, 1015µg, 1020µg, 1025µg, 1030µg, 1035µg, 1040µg, 1045µg, 1050µg, 1055µg, 1060µg, 1065µg, 1070µg, 1075µg, 1080µg, 1085µg, 1090µg, 1095µg, 1100µg, 1105µg, 1110µg, 1115µg, 1120µg, 1125µg, 1130µg, 1135µg, 1140µg, 1145µg, 1150µg, 1155µg, 1160µg, 1165µg, 1170µg, 1175µg, 1180µg, 1185µg, 1190µg, 1195µg, 1200µg, 1205µg, 1210µg, 1215µg, 1220µg, 1225µg, 1230µg, 1235µg, 1240µg, 1245µg, 1250µg, 1255µg, 1260µg, 1265µg, 1270µg, 1275µg, 1280µg, 1285µg, 1290µg, 1295µg, 1300µg, 1305µg, 1310µg, 1315µg, 1320µg, 1325µg, 1330µg, 1335µg, 1340µg, 1345µg, 1350µg, 1355µg, 1360µg, 1365µg, 1370µg, 1375µg, 1380µg, 1385µg, 1390µg, 1395µg, 1400µg, 1405µg, 1410µg, 1415µg, 1420µg, 1425µg, 1430µg, 1435µg, 1440µg, 1445µg, 1450µg, 1455µg, 1460µg, 1465µg, 1470µg, 1475µg, 1480µg, 1485µg, 1490µg, 1495µg, 1500µg, 1505µg, 1510µg, 1515µg, 1520µg, 1525µg, 1530µg, 1535µg, 1540µg, 1545µg, 1550µg, 1555µtg, 1560µg, 1565µg, 1570µg, 1575µg, 1580µg, 1585µg, 1590µg, 1595µg, 1600µg, 1605µg, 1610µg, 1615µg, 1620µg, 1625µg, 1630µg, 1635µg, 1640µg, 1645µg, 1650µg, 1655µg, 1660µg, 1665µg, 1670µg, 1675µg, 1680µg, 1685µg, 1690µg, 1695µg, 1700µg, 1705µg, 1710µg, 1715µg, 1720µg, 1725µg, 1730µg, 1735µg, 1740µg, 1745µg, 1750µg, 1755µg, 1760µg, 1765µg, 1770µg, 1775µg, 1780µg, 1785µg, 1790µg, 1795µg, 1800µg, 1805µg, 1810µg, 1815µg, 1820µg, 1825µg, 1830µg, 1835µg, 1840µg, 1845µg, 1850µg, 1855µg, 1860µg, 1865µg, 1870µg, 1875µg, 1880µg, 1885µg, 1890µg, 1895µg, 1900µg, 1905µg, 1910µg, 1915µg, 1920µg, 1925µg, 1930µg, 1935µg, 1940µg, 1945µg, 1950µg, 1955µg, 1960µg, 1965µg, 1970µg, 1975µg, 1980µg, 1985µg, 1990µg, 1995µg, 2000µg or similar. The suggested dosages per single dose specified above are not to be regarded as being limited to the numerical values actually stated, but are intended as dosages which are disclosed by way of example. Of course, dosages which may fluctuate about the abovementioned numerical values within a range of about +/- 2.5 µg are also included in the values given above by way of example. In these dosage ranges, the active substances **1** and **2** may be present in the weight ratios given above.

For example, without restricting the scope of the invention thereto, the combinations of **1** and **2** according to the invention may contain a quantity of **1** (if for instance the bromide is used as the preferred combination partner) and PDE-IV inhibitor **2** (as for instance ariflo, roflumilast or AWD-12-281) in such an amount that the following quantities of the active substances are administered per single dose: 40µg of **1** and 25µg of **2**, 40µg of **1** and 50µg of **2,** 40µg of **1** and 100µg of **2,** 40µg of **1** and 200µg of **2,** 40µg of **1** and 300µg **of 2,** 40µg of **1** and 400µg of **2**, 40µg of **1** and 500µg of **2**, 40µg of **1** and 600µg of **2,** 40µg of **1** and 700µg of **2,** 40µg of **1** and 800µg of **2**, 40µg of **1** and 900µg of **2**, 40µg of **1** and 1000µg of **2,** 60µg of **1** and 25µg **of 2,** 60µg of **1** and 50µg **of 2,** 60µg of **1** and 100µg **of 2,** 60µg of **1** and 200µg of **2**, 60µg of **1** and 300µg of **2**, 60µg of **1** and 400µg **of 2,** 60µg of **1** and 500µg of **2,** 60µg of **1** and 600µg of **2,** 60µg of **1** and 700µg of **2,** 60µg of **1** and 800µg of **2,** 60µg of **1** and 900µg of **2**, 60µg of **1** and 1000µg of **2**, 80µg of **1** and 25µg of **2**, 80µg of **1** and 50µg of **2**, 80µg of **1** and 100µg of **2**, 80µg of **1** and 200µg of **2**, 80µg of **1** and 300µg of **2**, 80µg of **1** and 400µg of **2,** 80µg of **1** and 500µg of **2,** 80µg of **1** and 600µg of **2**, 80µg of **1** and 700µg of **2**, 80µg of **1** and 800µg of **2**, 80µg of **1** and 900µg of **2**, 80µg of **1** and 1000µg of **2**, 100µg of **1** and 25µg of **2**, 100µg of **1** and 50µg of **2**, 100µg of **1** and 100µg of **2**, 100µg of **1** and 200µg of **2**, 100µg of **1** and 300µg of **2**, 100µg of **1** and 400µg of **2**, 100µg of **1** and 500µg of **2**, 100µg of **1** and 600µg of **2**, 100µg of **1** and 700µg of **2**, 100µg of **1** and 800µg of **2**, 100µg of **1** and 900µg of **2**, 100µg of **1** and 1000µg of **2**, 150µg of **1** and 25µg of **2**, 150µg of **1** and 50µg of **2**, 150µg of **1** and 100µg of **2**, 150µg of **1** and 200µg of **2**, 150µg of **1** and 300µg of **2**, 150µg of **1** and 400µg of **2**, 150µg of **1** and 500µg of **2**, 150µg of **1** and 600µg of **2**, 150µg of **1** and 700µg of **2**, 150µg of **1** and 800µg of **2**, 150µg of **1** and 900µg of **2**, 150µg of **1** and 1000µg of **2**, 200µg of **1** and 25µg of **2**, 200µg of **1** and 50µg of **2**, 200µg of **1** and 100µg of **2**, 200µg of **1** and 200µg of **2**, 200µg of **1** and 300µg of **2**, 200µg of **1** and 400µg of **2**, 200µg of **1** and 500µg of **2**, 200µg of **1** and 600µg of **2**, 200µg of **1** and 700µg of **2**, 200µg of **1** and 800µg of **2**, 200µg of **1** and 900µg of **2**, 200µg of **1** and 1000µg of **2**, 250µg of **1** and 25µg of **2**, 250µg of **1** and 50µg of **2**, 250µg of **1** and 100µg of **2**, 250µg of **1** and 200µg of **2**, 250µg of **1** and 300µg of **2**, 250µg of **1** and 400µg of **2**, 250µg of **1** and 500µg of **2**, 250µg of **1** and 600µg of **2**, 250µg of **1** and 700µg of **2**, 250µg of **1** and 800µg of **2**, 250µg of **1** and 900µg of **2**, 250µg of **1** and 1000µg of **2**, 400µg of **1** and 25µg of **2**, 400µg of **1** and 50µg of **2**, 400µg of **1** and 100µg of **2**, 400µg of **1** and 200µg of **2**, 400µg of **1** and 300µg of **2**, 400µg of **1** and 400µg of **2**, 400µg of **1** and 500µg of **2**, 400µg of **1** and 600µg of **2**, 400µg of **1** and 700µg of **2**, 400µg of **1** and 800µg of **2**, 400µg of **1** and 900µg of **2** or 400µg of **1** and 1000µg of **2**, 500µg of **1** and 25µg of **2**, 500µg of **1** and 50µg of **2**, 500µg of **1** and 100µg of **2**, 500µg of **1** and 200µg of **2**, 500µg of **1** and 300µg of **2**, 500µg of **1** and 400µg of **2**, 500µg of **1** and 500µg of **2**, 500µg of **1** and 600µg of **2**, 500µg of **1** and 700µg of **2**, 500µg of **1** and 800µg of **2**, 500µg of **1** and 900µg of **2** or 500µg of **1** and 1000µg of **2**, 600µg of **1** and 25µg of **2**, 600µg of **1** and 50µg of **2**, 600µg of **1** and 100µg of **2**, 600µg of **1** and 200µg of **2**, 600µg of and 300µg of **2**, 600µg of **1** and 400µg of **2**, 600µg of **1** and 500µg of **2**, 600µg of **1** and 600µg of **2**, 600µg of **1** and 700µg of **2**, 600µg of **1** and 800µg of **2**, 600µg of **1** and 900µg of **2**, 600µg-of **1** and 1000µg of **2**, 700µg of **1** and 25µg of **2**, 700µg of **1** and 50µg of **2**, 700µg of **1** and 100µg of **2**, 700µg of **1** and 200µg of **2**, 700µg of **1** and 300µg of **2**, 700µg of **1** and 400µg of **2**, 700µg of **1** and 500µg of **2**, 700µg of **1** and 600µg of **2**, 700µg of **1** and 700µg of **2**, 700µg of **1** and 800µg of **2**, 700µg of **1** and 900µg of **2**, 700µg of **1** and 1000µg of **2**, 800µg of **1** and 25µg of **2**, 800µg of **1** and 50µg of **2**, 800µg of **1** and 100µg of **2**, 800µg of **1** and 200µg of **2**, 800µg of **1** and 300µg of **2**, 800µg of **1** and 400µg of **2**, 800µg of **1** and 500µg of **2**, 800µg of **1** and 600µg of **2**, 800µg of **1** and 700µg of **2**, 800µg of **1** and 800µg of **2**, 800µg of **1** and 900µg of **2**, 800µg of **1** and 1000µg of **2**, 900µg of **1** and 25µg of **2**, 900µg of **1** and 50µg of **2**, 900µg of **1** and 100µg of **2**, 900µg of **1** and 200µg of **2**, 900µg of **1** and 300µg of **2**, 900µg of **1** and 400µg of **2**, 900µg of **1** and 500µg of **2**, 900µg of **1** and 600µg of **2**, 900µg of **1** and 700µg of **2**, 900µg of **1** and 800µg of **2**, 900µg of **1** and 900µg of **2**, 900µg of **1** and 1000µg of **2**, 1000µg of **1** and 25µg of **2**, 1000µg of **1** and 50µg of **2**, 1000µg of **1** and 100µg of **2**, 1000µg of **1** and 200µg of **2**, 1000µg of **1** and 300µg of **2**, 1000µg of **1** and 400µg of **2**, 1000µg of **1** and 500µg of **2**, 1000µg of **1** and 600µg of **2**, 1000µg of **1** and 700µg of **2**, 1000µg of 1 and 800µg of 2, 1000µg of **1** and 900µg of **2** or 1000µg of **1** and 1000µg of **2.**

The active substance combinations of **1** and **2** according to the invention are preferably administered by inhalation. For this purpose, ingredients **1** and **2** have to be made available in forms suitable for inhalation. Inhalable preparations according to the invention include inhalable powders, propellant-containing metered dose aerosols or propellant-free inhalable solutions. Inhalable powders according to the invention containing the combination of active substances **1** and **2** may consist of the active substances on their own or of a mixture of the active substances with physiologically acceptable excipients. Within the scope of the present invention, the term carrier may optionally be used instead of the term excipient. Within the scope of the present invention, the term propellant-free inhalable solutions also includes concentrates or sterile inhalable solutions ready for use. The preparations according to the invention may contain the combination of active substances **1** and **2** either together in one formulation or in two separate formulations. These formulations which may be used within the scope of the present invention are described in more detail in the next part of the specification.

### A) Inhalable powder containing the combinations of active substances 1 and 2 according to the invention:

The inhalable powders according to the invention may contain **1** and **2** either on their own or in admixture with suitable physiologically acceptable excipients.
If the active substances **1** and **2** are present in admixture with physiologically acceptable excipients, the following physiologically acceptable excipients may be used to prepare these inhalable powders according to the invention: monosaccharides (e.g. glucose or arabinose), disaccharides (e.g. lactose, saccharose, maltose, trehalose), oligo- and polysaccharides (e.g. dextran), polyalcohols (e.g. sorbitol, mannitol, xylitol), cyclodextrines (e.g. α-cyclodextrine, β-cyclodextrine, χ-cyclodextrine, methyl-β-cyclodextrine, hydroxypropyl-β-cyclodextrine), salts (e.g. sodium chloride, calcium carbonate) or mixtures of these excipients with one another. Preferably, mono- or disaccharides are used, while the use of lactose, trehalose or glucose is preferred, particularly, but not exclusively, in the form of their hydrates.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipient mentioned above. These finer excipients are also selected from the group of possible excipients listed hereinbefore. Finally, in order to prepare the inhalable powders according to the invention, micronised active substance **1** and **2**, preferably with an average particle size of 0.5 to 10µm, more preferably from 1 to 6µm, is added to the excipient mixture. Processes for producing the inhalable powders according to the invention by grinding and micronising and by finally mixing the ingredients together are known from the prior art. The inhalable powders according to the invention may be prepared and administered either in the form of a single powder mixture which contains both **1** and **2** or in the form of separate inhalable powders which contain only **1** or **2**.

The inhalable powders according to the invention may be administered using inhalers known from the prior art. Inhalable powders according to the invention which contain one or more physiologically acceptable excipients in addition to **1** and **2** may be administered, for example, by means of inhalers which deliver a single dose from a supply using a measuring chamber as described in US 4570630A, or by other means as described in DE 36 25 685 A. The inhalable powders according to the invention which contain **1** and **2** optionally in conjunction with a physiologically acceptable excipient may be administered, for example, using the inhaler known by the name Turbuhaler® or using inhalers as disclosed for example in EP 237507 A. Preferably, the inhalable powders according to the invention which contain physiologically acceptable excipient in addition to **1** and **2** are packed into capsules (to produce so-called inhalettes) which are used in inhalers as described, for example, in WO 94/28958.

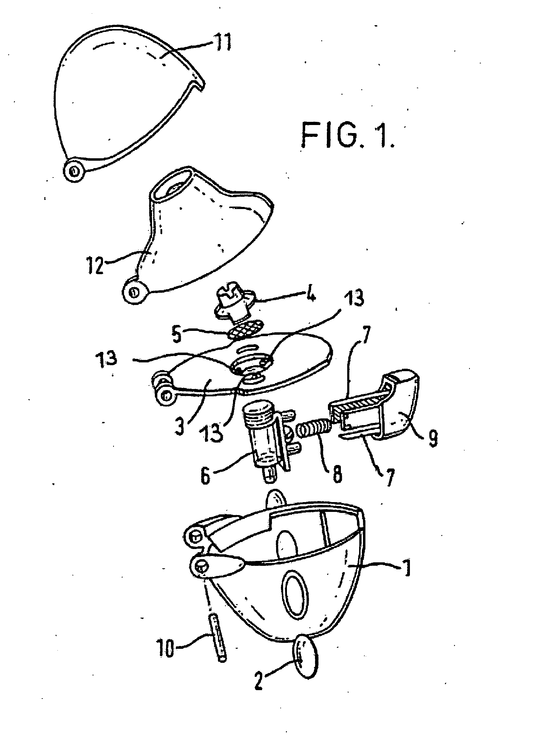

A particularly preferred inhaler for using the pharmaceutical combination according to the invention in inhalettes is shown in Figure 1.
This inhaler (Handihaler) for inhaling powdered pharmaceutical compositions from capsules is characterised by a housing 1 containing two windows 2, a deck 3 in which there are air inlet ports and which is provided with a screen 5 secured via a screen housing 4, an inhalation chamber 6 connected to the deck 3 on which there is a push button 9 provided with two sharpened pins 7 and movable counter to a spring 8, and a mouthpiece 12 which is connected to the housing 1, the deck 3 and a cover 11 via a spindle 10 to enable it to be flipped open or shut, as well as airholes 13 for adjusting the flow resistance.

If the inhalable powders according to the invention are packed into capsules (inhalers) for the preferred use described above, the quantities packed into each capsule should be 1 to 30mg per capsule. These capsules contain, according to the invention, either together or separately, the doses of **1** or **2** mentioned hereinbefore for each single dose.

### B) Propellant gas-driven inhalation aerosols containing the combinations of active substances 1 and 2:

Inhalation aerosols containing propellant gas according to the invention may contain substances **1** and **2** dissolved in the propellant gas or in dispersed form. **1** and **2** may be present in separate formulations or in a single preparation, in which **1** and **2** are either both dissolved, both dispersed or only one component is dissolved and the other is dispersed. The propellant gases which may be used to prepare the inhalation aerosols according to the invention are known from the prior art. Suitable propellant gases are selected from among hydrocarbons such as n-propane, n-butane or isobutane and halohydrocarbons such as fluorinated derivatives of methane, ethane, propane, butane, cyclopropane or cyclobutane. The propellant gases mentioned above may be used on their own or in mixtures thereof. Particularly preferred propellant gases are halogenated alkane derivatives selected from TG11, TG12, TG134a (1,1,1,2-tetrafluoroethane) and TG227 (1,1,1,2,3,3,3-heptafluoropropane) and mixtures thereof, of which the propellant gases TG134a, TG227 and mixtures thereof are preferred.

The propellant-driven inhalation aerosols according to the invention may also contain other ingredients such as co-solvents, stabilisers, surfactants, antioxidants, lubricants and pH adjusters. All these ingredients are known in the art.

The inhalation aerosols containing propellant gas according to the invention may contain up to 5 wt.-% of active substance **1** and/or **2**. Aerosols according to the invention contain, for example, 0.002 to 5 wt.-%, 0.01 to 3 wt.-%, 0.015 to 2 wt.-%, 0.1 to 2 wt.-%, 0.5 to 2 wt.-% or 0.5 to 1 wt.-% of active substance **1** and/or **2**.

If the active substances **1** and/or **2** are present in dispersed form, the particles of active substance preferably have an average particle size of up to 10µm, preferably from 0.1 to 6µm, more preferably from 1 to 5µm.

The propellant-driven inhalation aerosols according to the invention mentioned above may be administered using inhalers known in the art (MDIs = metered dose inhalers). Accordingly, in another aspect, the present invention relates to pharmaceutical compositions in the form of propellant-driven aerosols as hereinbefore described combined with one or more inhalers suitable for administering these aerosols. In addition, the present invention relates to inhalers which are characterised in that they contain the propellant gas-containing aerosols described above according to the invention. The present invention also relates to cartridges fitted with a suitable valve which can be used in a suitable inhaler and which contain one of the above-mentioned propellant gas-containing inhalation aerosols according to the invention. Suitable cartridges and methods of filling these cartridges with the inhalable aerosols containing propellant gas according to the invention are known from the prior art.

### C) Propellant-free inhalable solutions or suspensions containing the combinations of active substances 1 and 2 according to the invention:

Propellant-free inhalable solutions and suspensions according to the invention contain, for example, aqueous or alcoholic, preferably ethanolic solvents, optionally ethanolic solvents mixed with aqueous solvents. If aqueous/ethanolic solvent mixtures are used the relative proportion of ethanol compared with water is not limited but preferably the maximum is up to 70 percent by volume, more particularly up to 60 percent by volume of ethanol. The remainder of the volume is made up of water. The solutions or suspensions containing **1** and **2**, separately or together, are adjusted to a pH of 2 to 7, preferably 2 to 5, using suitable acids. The pH may be adjusted using acids selected from inorganic or organic acids. Examples of particularly suitable inorganic acids include hydrochloric acid, hydrobromic acid, nitric acid, sulphuric acid and/or phosphoric acid. Examples of particularly suitable organic acids include ascorbic acid, citric acid, malic acid, tartaric acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g. as flavourings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium editate, as stabiliser or complexing agent is unnecessary in the present formulation. Other embodiments may contain this compound or these compounds. In a preferred embodiment the content based on sodium editate is less than 100mg/100ml, preferably less than 50mg/100 ml, more preferably less than 20mg/100 ml. Generally, inhalable solutions in which the content of sodium editate is from 0 to 10mg/100ml are preferred.

Co-solvents and/or other excipients may be added to the propellant-free inhalable solutions according to the invention. Preferred co-solvents are those which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation. Preferably, these substances have no pharmacological effect or, in connection with the desired therapy, no appreciable or at least no undesirable pharmacological effect. The excipients and additives include, for example, surfactants such as soya lecithin, oleic acid, sorbitan esters, such as polysorbates, polyvinylpyrrolidone, other stabilisers, complexing agents, antioxidants and/or preservatives which guarantee or prolong the shelf life of the finished pharmaceutical formulation, flavourings, vitamins and/or other additives known in the art. The additives also include pharmacologically acceptable salts such as sodium chloride as isotonic agents. The preferred excipients include antioxidants such as ascorbic acid, for example, provided that it has not already been used to adjust the pH, vitamin A, vitamin E, tocopherols and similar vitamins and provitamins occurring in the human body.

Preservatives may be used to protect the formulation from contamination with pathogens. Suitable preservatives are those which are known in the art, particularly cetyl pyridinium chloride, benzalkonium chloride or benzoic acid or benzoates such as sodium benzoate in the concentration known from the prior art. The preservatives mentioned above are preferably present in concentrations of up to 50mg/100ml, more preferably between 5 and 20mg/100ml.

Preferred formulations contain, in addition to the solvent water and the combination of active substances **1** and **2**, only benzalkonium chloride and sodium editate. In another preferred embodiment, no sodium editate is present.

The propellant-free inhalable solutions according to the invention are administered in particular using inhalers of the kind which are capable of nebulising a small amount of a liquid formulation in the therapeutic dose within a few seconds to produce an aerosol suitable for therapeutic inhalation. Within the scope of the present invention, preferred inhalers are those in which a quantity of less than 100µL, preferably less than 50µL, more preferably between 20 and 30µL of active substance solution can be nebulised in preferably one spray action to form an aerosol with an average particle size of less than 20µm, preferably less than 10µm, in such a way that the inhalable part of the aerosol corresponds to the therapeutically effective quantity.

An apparatus of this kind for propellant-free delivery of a metered quantity of a liquid pharmaceutical composition for inhalation is described for example in International Patent Application WO 91/14468 and also in WO 97/12687 (cf. in particular Figures 6a and 6b). The nebulisers (devices) described therein are known by the name Respimat®.
This nebuliser (Respimat®) can advantageously be used to produce the inhalable aerosols according to the invention containing the combination of active substances **1** and **2**. Because of its cylindrical shape and handy size of less than 9 to 15 cm long and 2 to 4 cm wide, this device can be carried at all times by the patient. The nebuliser sprays a defined volume of pharmaceutical formulation using high pressures through small nozzles so as to produce inhalable aerosols.

The preferred atomiser essentially consists of an upper housing part, a pump housing, a nozzle, a locking mechanism, a spring housing, a spring and a storage container, characterised by
- a pump housing which is secured in the upper housing part and which comprises at one end a nozzle body with the nozzle or nozzle arrangement,
- a hollow plunger with valve body,
- a power takeoff flange in which the hollow plunger is secured and which is located in the upper housing part,
- a locking mechanism situated in the upper housing part,
- a spring housing with the spring contained therein, which is rotatably mounted on the upper housing part by means of a rotary bearing,
- a lower housing part which is fitted onto the spring housing in the axial direction.

The hollow plunger with valve body corresponds to a device disclosed in WO 97/12687. It projects partially into the cylinder of the pump housing and is axially movable within the cylinder. Reference is made in particular to Figures 1 to 4, especially Figure 3, and the relevant parts of the description. The hollow plunger with valve body exerts a pressure of 5 to 60 Mpa (about 50 to 600 bar), preferably 10 to 60 Mpa (about 100 to 600 bar) on the fluid, the measured amount of active substance solution, at its high pressure end at the moment when the spring is actuated. Volumes of 10 to 50 microlitres are preferred, while volumes of 10 to 20 microlitres are particularly preferred and a volume of 15 microlitres per spray is most particularly preferred.

The valve body is preferably mounted at the end of the hollow plunger facing the valve body.

The nozzle in the nozzle body is preferably microstructured, i.e. produced by microtechnology. Microstructured nozzle bodies are disclosed for example in WO 94/07607; reference is hereby made to the contents of this specification, particularly Figure 1 therein and the associated description.
The nozzle body consists for example of two sheets of glass and/or silicon firmly joined together, at least one of which has one or more microstructured channels which connect the nozzle inlet end to the nozzle outlet end. At the nozzle outlet end there is at least one round or non-round opening 2 to 10 microns deep and 5 to 15 microns wide, the depth preferably being 4.5 to 6.5 microns while the length is preferably 7 to 9 microns.
In the case of a plurality of nozzle openings, preferably two, the directions of spraying of the nozzles in the nozzle body may extend parallel to one another or may be inclined relative to one another in the direction of the nozzle opening. In a nozzle body with at least two nozzle openings at the outlet end the directions of spraying may be at an angle of 20 to 160° to one another, preferably 60 to 150°, most preferably 80 to 100°. The nozzle openings are preferably arranged at a spacing of 10 to 200 microns, more preferably at a spacing of 10 to 100 microns, most preferably 30 to 70 microns. Spacings of 50 microns are most preferred. The directions of spraying will therefore meet in the vicinity of the nozzle openings.
The liquid pharmaceutical preparation strikes the nozzle body with an entry pressure of up to 600 bar, preferably 200 to 300 bar, and is atomised into an inhalable aerosol through the nozzle openings. The preferred particle or droplet sizes of the aerosol are up to 20 microns, preferably 3 to 10 microns.

The locking mechanism contains a spring, preferably a cylindrical helical compression spring, as a store for the mechanical energy. The spring acts on the power takeoff flange as an actuating member the movement of which is determined by the position of a locking member. The travel of the power takeoff flange is precisely limited by an upper and lower stop. The spring is preferably biased, via a power step-up gear, e.g. a helical thrust gear, by an external torque which is produced when the upper housing part is rotated counter to the spring housing in the lower housing part. In this case, the upper housing part and the power takeoff flange have a single or multiple V-shaped gear.

The locking member with engaging locking surfaces is arranged in a ring around the power takeoff flange. It consists, for example, of a ring of plastic or metal which is inherently radially elastically deformable. The ring is arranged in a plane at right angles to the atomiser axis. After the biasing of the spring, the locking surfaces of the locking member move into the path of the power takeoff flange and prevent the spring from relaxing. The locking member is actuated by means of a button. The actuating button is connected or coupled to the locking member. In order to actuate the locking mechanism, the actuating button is moved parallel to the annular plane, preferably into the atomiser; this causes the deformable ring to deform in the annular plane. Details of the construction of the locking mechanism are given in WO 97/20590.

The lower housing part is pushed axially over the spring housing and covers the mounting, the drive of the spindle and the storage container for the fluid.
When the atomiser is actuated the upper housing part is rotated relative to the lower housing part, the lower housing part taking the spring housing with it. The spring is thereby compressed and biased by means of the helical thrust gear and the locking mechanism engages automatically. The angle of rotation is preferably a whole-number fraction of 360 degrees, e.g. 180 degrees. At the same time as the spring is biased, the power takeoff part in the upper housing part is moved along by a given distance, the hollow plunger is withdrawn inside the cylinder in the pump housing, as a result of which some of the fluid is sucked out of the storage container and into the high pressure chamber in front of the nozzle.

If desired, a number of exchangeable storage containers which contain the fluid to be atomised may be pushed into the atomiser one after another and used in succession. The storage container contains the aqueous aerosol preparation according to the invention. The atomising process is initiated by pressing gently on the actuating button. As a result, the locking mechanism opens up the path for the power takeoff member. The biased spring pushes the plunger into the cylinder of the pump housing. The fluid leaves the nozzle of the atomiser in atomised form.

Further details of construction are disclosed in PCT Applications WO 97/12683 and WO 97/20590, to which reference is hereby made.
The components of the atomiser (nebuliser) are made of a material which is suitable for its purpose. The housing of the atomiser and, if its operation permits, other parts as well, are preferably made of plastics, e.g. by injection moulding. For medicinal purposes, physiologically safe materials are used.

Figures 6a/b of WO 97/12687, show the nebuliser (Respimat®) which can advantageously be used for inhaling the aqueous aerosol preparations according to the invention.
Figure 6a of WO 97/12687 shows a longitudinal section through the atomiser with the spring biased while Figure 6b of WO 97/12687 shows a longitudinal section through the atomiser with the spring relaxed.
The upper housing part (51) contains the pump housing (52) on the end of which is mounted the holder (53) for the atomiser nozzle. In the holder is the nozzle body (54) and a filter (55). The hollow plunger (57) fixed in the power takeoff flange (56) of the locking mechanism projects partially into the cylinder of the pump housing. At its end the hollow plunger carries the valve body (58). The hollow plunger is sealed off by means of the seal (59). Inside the upper housing part is the stop (60) on which the power takeoff flange abuts when the spring is relaxed. On the power takeoff flange is the stop (61) on which the power takeoff flange abuts when the spring is biased. After the biasing of the spring the locking member (62) moves between the stop (61) and a support (63) in the upper housing part. The actuating button (64) is connected to the locking member. The upper housing part ends in the mouthpiece (65) and is sealed off by means of the protective cover (66) which can be placed thereon.
The spring housing (67) with compression spring (68) is rotatably mounted on the upper housing part by means of the snap-in lugs (69) and rotary bearing. The lower housing part (70) is pushed over the spring housing. Inside the spring housing is the exchangeable storage container (71) for the fluid (72) which is to be atomised. The storage container is sealed off by the stopper (73) through which the hollow plunger projects into the storage container and is immersed at its end in the fluid (supply of active substance solution).
The spindle (74) for the mechanical counter is mounted in the covering of the spring housing. At the end of the spindle facing the upper housing part is the drive pinion (75). The slider (76) sits on the spindle.

The nebuliser described above is suitable for nebulising the aerosol preparations according to the invention to produce an aerosol suitable for inhalation.
If the formulation according to the invention is nebulised using the method described above (Respimat®) the quantity delivered should correspond to a defined quantity with a tolerance of not more than 25%, preferably 20% of this amount in at least 97%, preferably at least 98% of all operations of the inhaler (spray actuations). Preferably, between 5 and 30 mg of formulation, most preferably between 5 and 20 mg of formulation are delivered as a defined mass on each actuation.

However, the formulation according to the invention may also be nebulised by means of inhalers other than those described above, e.g. jet stream inhalers or other stationary nebulisers.

Accordingly, in a further aspect, the invention relates to pharmaceutical formulations in the form of propellant-free inhalable solutions or suspensions as described above combined with a device suitable for administering these formulations, preferably in conjunction with the Respimat®. Preferably, the invention relates to propellant-free inhalable solutions or suspensions characterised by the combination of active substances **1** and **2** according to the invention in conjunction with the device known by the name Respimat®. In addition, the present invention relates to the above-mentioned devices for inhalation, preferably the Respimat®, characterised in that they contain the propellant-free inhalable solutions or suspensions according to the invention as described hereinbefore.

According to the invention, inhalable solutions which contain the active substances 1 and 2 in a single preparation are preferred. The term "single preparation" also includes preparations which contain the two ingredients **1** and **2** in two-chamber cartridges, as disclosed for example in WO 00/23037. Reference is hereby made to this publication in its entirety.

The propellant-free inhalable solutions or suspensions according to the invention may take the form of concentrates or sterile inhalable solutions or suspensions ready for use, as well as the above-mentioned solutions and suspensions designed for use in a Respimat®. Formulations ready for use may be produced from the concentrates, for example, by the addition of isotonic saline solutions. Sterile formulations ready for use may be administered using energy-operated free-standing or portable nebulisers which produce inhalable aerosols by means of ultrasound or compressed air by the Venturi principle or other principles.

Accordingly, in another aspect, the present invention relates to pharmaceutical compositions in the form of propellant-free inhalable solutions or suspensions as described hereinbefore which take the form of concentrates or sterile formulations ready for use, combined with a device suitable for administering these solutions, characterised in that the device is an energy-operated free-standing or portable nebuliser which produces inhalable aerosols by means of ultrasound or compressed air by the Venturi principle or other methods.

The Examples which follow serve to illustrate the present invention in more detail without restricting the scope of the invention to the following embodiments by way of example.

### Examples of Formulations

The following examples of formulations, which may be obtained analogously to methods known in the art, serve to illustrate the present invention more fully without restricting it to the contents of these examples.

### Inhalable powders:

1)

| **Ingredients** | **µg per capsule** |
|---|---|
| **1** (bromide, racemate) | 60 |
| AWD-12-281 | 200 |
| lactose | 12240 |
| **Total** | 12500 |

2)

| **Ingredients** | **µg per capsule** |
|---|---|
| **1** (bromide, racemate) | 20 |
| AWD-12-281 | 100 |
| lactose | 12380 |
| **Total** | 12500 |

3)

| **Ingredients** | **µg per capsule** |
|---|---|
| **1** (bromide, racemate) | 60 |
| AWD-12-281 | 300 |
| lactose | 12140 |
| **Total** | 12500 |

4)

| **Ingredients** | **µg per capsule** |
|---|---|
| **1** (bromide, racemate) | 60 |
| ariflo | 200 |
| lactose | 12240 |
| **Total** | 12500 |

5)

| **Ingredients** | **µg per capsule** |
|---|---|
| **1** (bromide, racemate) | 20 |
| ariflo | 100 |
| lactose | 12380 |
| **Total** | 12500 |

6)

| **Ingredients** | **µg per capsule** |
|---|---|
| **1** (bromide, racemate) | 60 |
| ariflo | 300 |
| lactose | 12140 |
| **Total** | 12500 |

7)

| **Ingredients** | **µg per capsule** |
|---|---|
| **1** (bromide, racemate) | 60 |
| roflumilast | 200 |
| lactose | 12240 |
| **Total** | 12500 |

8)

| **Ingredients** | **µg per capsule** |
|---|---|
| **1** (bromide, racemate) | 20 |
| roflumilast | 100 |
| lactose | 12380 |
| **Total** | 12500 |

9)

| **Ingredients** | **µg per capsule** |
|---|---|
| **1** (bromide, racemate) | 60 |
| roflumilast | 300 |
| lactose | 12140 |
| **Total** | 12500 |

10)

| | |
|---|---|
| **Ingredients** | **µg per capsule** |
| **1** (bromide, racemate) | 60 |
| Z-15370 | 200 |
| lactose | 12240 |
| **Total** | 12500 |

11)

| **Ingredients** | **µg per capsule** |
|---|---|
| **1** (bromide, racemate) | 20 |
| Z-15370 | 200 |
| lactose | 12280 |
| **Total** | 12500 |

12)

| **Ingredients** | **µg per capsule** |
|---|---|
| **1** (bromide, racemate) | 60 |
| Z-15-370 | 200 |
| lactose | 12240 |
| **Total** | 12500 |

13)

| **Ingredients** | **µg per capsule** |
|---|---|
| (**2'R**,**3R**) **1** (bromide) | 60 |
| AWD-12-281 | 200 |
| lactose | 12240 |
| **Total** | 12500 |

14)

| **Ingredients** | **µg per capsule** |
|---|---|
| **(2'R,3R) 1** (bromide) | 20 |
| AWD-12-281 | 100 |
| lactose | 12380 |
| **Total** | 12500 |

15)

| **Ingredients** | **µg per capsule** |
|---|---|
| (**2'R**,**3R**) **1** (bromide) | 60 |
| AWD-12-281 | 300 |
| lactose | 12140 |
| **Total** | 12500 |

16)

| **Ingredients** | **µg per capsule** |
|---|---|
| (**2'R**,**3R**) **1** (bromide) | 60 |
| ariflo | 200 |
| lactose | 12240 |
| **Total** | 12500 |

17)

| **Ingredients** | **µg per capsule** |
|---|---|
| (**2'R**,**3R**) **1** (bromide) | 20 |
| ariflo | 100 |
| lactose | 12380 |
| **Total** | 12500 |

18)

| **Ingredients** | **µg per capsule** |
|---|---|
| (**2'R**,**3R**) **1** (bromide) | 60 |
| - ariflo | 300 |
| lactose | 12140 |
| **Total** | 12500 |

19)

| **Ingredients** | **µg per capsule** |
|---|---|
| (**2'R**,**3R**) **1** (bromide) | 60 |
| roflumilast | 200 |
| lactose | 12240 |
| **Total** | 12500 |

20)

| **Ingredients** | **µg per capsule** |
|---|---|
| (**2'R**,**3R**) **1** (bromide) | 20 |
| roflumilast | 100 |
| lactose | 12380 |
| **Total** | 12500 |

21)

| **Ingredients** | **µg per capsule** |
|---|---|
| (**2'R**,**3R**) **1** (bromide) | 60 |
| roflumilast | 300 |
| lactose | 12140 |
| **Total** | 12500 |

22)

| **Ingredients** | **µg per capsule** |
|---|---|
| **(2'R,3R) 1** (bromide) | 60 |
| Z-15370 | 200 |
| lactose | 12240 |
| **Total** | 12500 |

23)

| **Ingredients** | **µg per capsule** |
|---|---|
| **(2'R,3R) 1** (bromide) | 20 |
| Z-15370 | 200 |
| lactose | 12280 |
| **Total** | 12500 |

24)

| **Ingredients** | **µg per capsule** |
|---|---|
| (**2'R**,**3R**) **1** (bromide) | 60 |
| Z-15-370 | 200 |
| lactose | 12240 |
| **Total** | 12500 |

Formulation examples 13 to 24 contain the diastereomer **(2'R, 3R) 1**. Examples containing **(2'S, 3R) 1**, **(2'S, 3S) 1**, **or (2'R, 3S) 1** instead are analogously obtainable.

### B) Propellant-containing aerosols for inhalation:

1)

| **Ingredients** | **% by weight** |
|---|---|
| **1** (bromide, racemate) | 0.050 |
| AWD-12-281 | 0.060 |
| soya lecithin | 0.2 |
| TG 134a : TG227 = 2:3 | ad 100 |

2)

| **Ingredients** | **% by weight** |
|---|---|
| **1** (bromide, racemate) | 0.050 |
| ariflo | 0.030 |
| Isopropyl myristate | 0.1 |
| TG 227 | ad 100 |

3)

| **Ingredients** | **% by weight** |
|---|---|
| **1** (bromide, racemate) | 0.050 |
| Z-15370 | 0.030 |
| Isopropyl myristate | 0.1 |
| TG 227 | ad 100 |

4)

| **Ingredients** | **% by weight** |
|---|---|
| **1** (bromide, racemate) | 0.2 |
| roflumilast | 0.4 |
| Isopropyl myristate | 0.1 |
| TG 134a : TG227 = 2:3 | ad 100 |

5)

| **Ingredients** | **% by weight** |
|---|---|
| (**2'R**,**3R**) **1** (bromide) | 0.050 |
| AWD-12-281 | 0.060 |
| soya lecithin | 0.2 |
| TG 134a : TG227 = 2:3 | ad 100 |

6)

| **Ingredients** | **% by weight** |
|---|---|
| **(2'R,3R) 1** (bromide) | 0.050 |
| ariflo | 0.030 |
| Isopropyl myristate | 0.1 |
| TG 227 | ad 100 |

7)

| **Ingredients** | **% by weight** |
|---|---|
| **(2'R,3R) 1** (bromide) | 0.050 |
| Z-15370 | 0.030 |
| Isopropyl myristate | 0.1 |
| TG 227 | ad 100 |

8)

| **Ingredients** | **% by weight** |
|---|---|
| (**2'R**,**3R**) **1** (bromide) | 0.2 |
| - roflumilast | 0.4 |
| Isopropyl myristate | 0.1 |
| TG 134a : TG227 = 2:3 | ad 100 |

Aerosol formulation examples 5 to 8 contain the diastereomer (**2'R**, **3R**) **1**. Examples containing **(2'S, 3R) 1**, **(2'S, 3S) 1**, **or (2'R, 3S) 1** instead are analogously obtainable.

## Claims

1. Pharmaceutical compositions, **characterised in that** they contain one or more salts of formula **1** 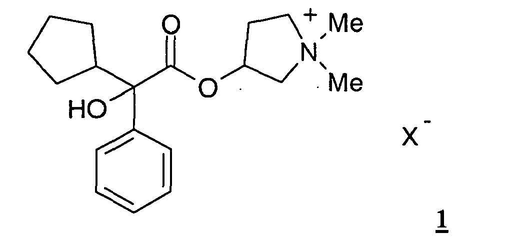
wherein
X - denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate, optionally in the form of the racemates, the stereoisomers, and the hydrates thereof,
combined with one or more PDE IV inhibitors (**2**), optionally in the form of the stereoisomers, mixtures of the stereoisomers or in the form of the racemates thereof, optionally in the form of the solvates or hydrates and optionally together with a pharmaceutically acceptable excipient.

2. Pharmaceutical composition according to claim 1, **characterised in that** the active substances **1** and **2** are present either together in a single formulation or in two separate formulations.

3. Pharmaceutical composition according to claim 1 or 2, **characterised in that** the compounds of formula **1** are present in form of the diastereoisomers of formula **(2R',3S) 1**

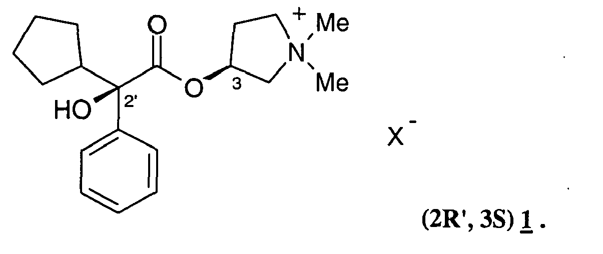

4. Pharmaceutical composition according to claim 1 or 2, **characterised in that** the compounds of formula **1** are present in form of the diastereoisomers of formula **(2'S,3S) 1**

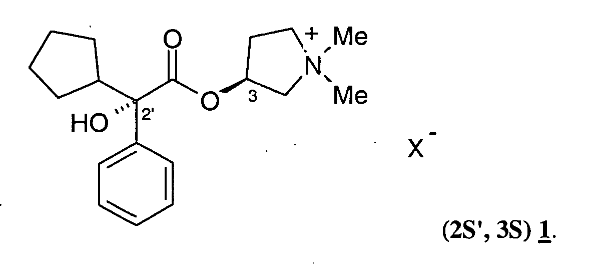

5. Pharmaceutical composition according to claim 1 or 2, **characterised in that** the compounds of formula **1** are present in form of the diastereoisomers of formula **(2'S,3R) 1**

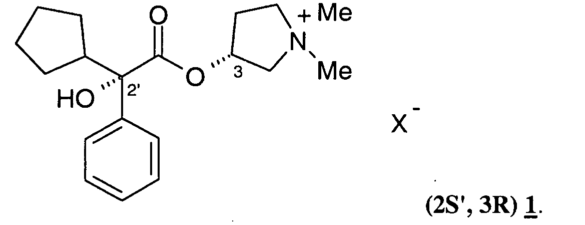

6. Pharmaceutical composition according to claim 1 or 2, **characterised in that** the compounds of formula **1** are present in form of the diastereoisomers of formula **(2'R,3R) 1**

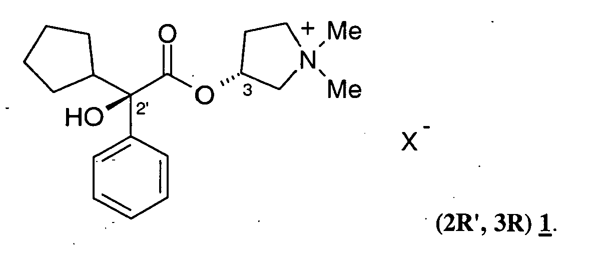

7. Pharmaceutical composition according to one of claims 1 to 6, **characterised in that** the PDE IV inhibitors **2** are selected from the group consisting of enprofylline, theophylline, roflumilast, ariflo (cilomilast), CP-325,366, BY343, D-4396 (Sch-351591), AWD-12-281 (GW-842470), N-(3,5-dichloro-1-oxo-pyridin-4-yl)-4-difluoromethoxy-3-cyclopropylmethoxybenzamide, NCS-613, pumafentine, (-)p-[(4aR*,10bS*)-9-ethoxy-1,2,3,4,4a,10b-hexahydro-8-methoxy-2methylbenzo[s][1,6]naphthyridin-6-yl]-N,N-diisopropylbenzamide, (R)-(+)-1-(4-bromobenzyl)-4-[(3-cyclopentyloxy)-4-methoxyphenyl]-2-pyrrolidone, 3-(cyclopentyloxy-4-methoxyphenyl)-1-(4-N'-[N-2-cyano-S-methyl-isothioureido]benzyl)-2-pyrrolidone, cis[4-cyano-4-(3-cyclopentyloxy-4-methoxyphenyl)cyclohexan-l-carboxylic acid], 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], (R)-(+)-ethyl[4-(3-cyclopentyloxy-4-methoxyphenyl)pyrrolidine-2-ylidene]acetate, (S)-(-)-ethyl[4-(3-cyclopentyloxy-4-methoxyphenyl)pyrrolidine-2-ylidene]acetate, CDP840, Bay-198004, D-4418, PD-168787, T-440, T-2585, arofylline, atizoram, V-11294A, C1-1018, CDC-801, CDC-3052, D-22888, YM-58997, Z-15370, 9-cyclopentyl-5,6-dihydro-7-ethyl-3-(2-thienyl)-9H-pyrazolo[3,4 c]-1,2,4-triazolo[4,3-a]pyridine, and 9-cyclopentyl-5,6-dihydro-7-ethyl-3-(tert-butyl)-9H-pyrazolo[3,4 c]-1,2,4-triazolo[4,3-a]pyridine, optionally in the form of the racemates, the enantiomers, the diastereomers and optionally the pharmacologically acceptable acid addition salts thereof, and the hydrates thereof.

8. Pharmaceutical compositions according to one of claims 1 to 7, **characterised in that** the compounds **2** are selected from the group consisting of enprofylline, roflumilast, ariflo (cilomilast), AWD-12-281 (GW-842470), N-(3,5-dichloro-1-oxo-pyridin-4-yl)-4-difluoromethoxy-3-cyclopropylmethoxybenzamide, T-440, T-2585, arofylline, cis[4-cyano-4-(3-cyclopentyloxy-4-methoxyphenyl)cyclohexan-1-carboxylic acid], 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], PD-168787, atizoram, V-11294A, Cl-1018, CDC-801, D-22888, YM-58997, Z-15370, 9-cyclopentyl-5,6-dihydro-7-ethyl-3-(2-thienyl)-9H-pyrazolo[3,4 c]-1,2,4-triazolo[4,3-a]pyridine, and 9-cyclopentyl-5,6-dihydro-7-ethyl-3-(tert-butyl)-9H-pyrazolo[3,4 c]-1,2,4-triazolo[4,3-a]pyridine, optionally in the form of the racemates, the enantiomers, the diastereomers and optionally the pharmacologically acceptable acid addition salts thereof, and the hydrates thereof.

9. Pharmaceutical compositions according to one of claims 1 to 8, **characterised in that** the compounds **2** are selected from among roflumilast, ariflo (cilomilast), AWD-12-281 (GW-842470), arofylline, Z-15370, 2-carbomethoxy-4-cyano-4-(3-cyclopropylmethoxy-4-difluoromethoxyphenyl)cyclohexan-1-one, cis[4-cyano-4-(3-cyclopropylmethoxy-4-difluoromethoxyphenyl)cyclohexan-1-ol], atizoram, 9-cyclopentyl-5,6-dihydro-7-ethyl-3-(2-thienyl)-9H-pyrazolo[3,4 c]-1,2,4-triazolo[4,3-a]pyridine, and 9-cyclopentyl-5,6-dihydro-7-ethyl-3-(tert-butyl)-9H-pyrazolo[3,4 c]-1,2,4-triazolo[4,3-a]pyridine, optionally in the form of the racemates, the enantiomers, the diastereomers and optionally the pharmacologically acceptable acid addition salts thereof, and the hydrates thereof.

10. Pharmaceutical compositions according to one of claims 1 to 9, **characterised in that** the weight ratios of 1 to 2 are in a range from about 1:100 to 100:1, preferably from 1:80 to 80:1.

11. Pharmaceutical composition according to one of claims 1 to 9, **characterised in that** it is in the form of a preparation suitable for inhalation.

12. Pharmaceutical composition according to claim 11, **characterised in that** it is a preparation selected from among the inhalable powders, propellant-containing metered-dose aerosols and propellant-free inhalable solutions.

13. Pharmaceutical composition according to claim 12, **characterised in that** it is an inhalable powder which contains 1 and 2 in admixture with suitable physiologically acceptable excipients selected from among the monosaccharides, disaccharides, oligo and polysaccharides, polyalcohols, salts, or mixtures of these excipients with one another.

14. Inhalable powder according to claim 13, **characterised in that** the excipient has a maximum average particle size of up to 250µm, preferably between 10 and 150µm.

15. Pharmaceutical composition according to claim 12, **characterised in that** it is an inhalable powder which contains only the active substances 1 and 2 as its ingredients.

16. Pharmaceutical composition according to claim 12, **characterised in that** it is a propellant containing inhalable aerosol which contains 1 and 2 in dissolved or dispersed form.

17. Propellant containing inhalable aerosol according to claim 16, **characterised in that** it contains, as propellant gas, hydrocarbons such as n propane, n butane or isobutane or halohydrocarbons such as chlorinated and/or fluorinated derivatives of methane, ethane, propane, butane, cyclopropane or cyclobutane.

18. Propellant containing inhalable aerosol according to claim 17, **characterised in that** the propellant gas is TG11, TG12, TG134a, TG227 or mixtures thereof, preferably TG134a, TG227 or a mixture thereof.

19. Propellant containing inhalable aerosol according to one of claims 16 to 18, **characterised in that** it may contain up to 5 % by weight of active substance 1 and/or 2.

20. Pharmaceutical composition according to claim 12, **characterised in that** it is a propellant free inhalable solution which contains water, ethanol or a mixture of water and ethanol as solvent.

21. Inhalable solution according to claim 20, **characterised in that** it optionally contains other co solvents and/or excipients.

22. Inhalable solution according to claim 21, **characterised in that** it contains as co solvents ingredients which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters.

23. Inhalable solutions according to one of claims 21 or 22, **characterised in that** they contain as excipients surfactants, stabilisers, complexing agents, antioxidants and/or preservatives, flavourings, pharmacologically acceptable salts and/or vitamins.

24. Inhalable solutions according to claim 23, **characterised in that** they contain as complexing agents editic acid or a salt of editic acid, preferably sodium edetate.

25. Use of a composition according to one of claims 1 to 24 for preparing a medicament for the treatment of inflammatory or obstructive respiratory complaints, particularly asthma or COPD.
